# EUROPEAN PATENT SPECIFICATION

(11) **EP 3 600 346 B1**
(45) Date of publication and mention of the grant of the patent: **09.12.2026**
(21) Application number: 18712022.5
(22) Date of filing: 19.03.2018
(51) Int. Cl.: A61K 31/714, A61K 36/68, A61P 3/02, A61P 25/28

(54) **NUTRACEUTICAL, DIETETIC AND NUTRITIONAL COMPOSITION WITH ANTIOXIDANT ACTIVITY**
NUTRAZEUTISCHE, DIÄTETISCHE UND ERNÄHRUNGSZUSAMMENSETZUNG MIT ANTIOXIDATIVER WIRKUNG
COMPOSITION NUTRACEUTIQUE, DIÉTÉTIQUE ET NUTRITIONNELLE À ACTIVITÉ ANTIOXYDANTE

(30) Priority: 23.03.2017 IT 201700031902
(43) Date of publication of application: 05.02.2020
(73) Proprietor: A. Menarini Industrie Farmaceutiche Riunite S.r.l., 50131 Firenze (IT)
(72) Inventor: MELANI, Francesco, 50014 Fiesole (FI) (IT)
(74) Representative: Di Giovine, Paolo
(86) International application number: PCT/IB2018/051812
(87) International publication number: WO 2018/172903

(56) References cited:
- WO-A1-2016/109856
- WO-A2-2016/109853
- US-A1- 2010 021 533
- US-A1- 2013 034 530

## Description

### FIELD OF THE INVENTION

The present invention relates to a combination and to a composition for oral use, in particular in the form of a nutraceutical, dietetic and nutritional composition comprising an extract of Bacopa, vitamin B12, lycopene and astaxanthin. The present invention also relates to the use of such a composition to improve cognitive performance and/or to improve mood and/or the state of psychological well-being.

### PRIOR ART

Oxidative stress is considered to be the common effector of the cascade of degenerative events in many neurological diseases. [Jenner P, Olanow CW "Oxidative stress and the pathogenesis of Parkinson's disease Neurology" 1996 Dec;47(6 Suppl 3):S161-70]. Neuronal cell death caused by oxidative stress and by mitochdonrial dysfunction is involved in the reduced cognitive activity associated with ageing and in neurodegenerative pathologies, such as Alzheimer's disease. The molecular mechanisms of neuronal degeneration remain largely unknown and are not currently available for effective therapies. Various studies have shown that antioxidants such as carotenoids and, in particular, lycopene, and flavonoids are potentially useful in the management of degenerative pathologies [Rao AV and Balachandran B. Role of Oxidative Stress and Antioxidants in Neurodegenerative Diseases Pages 291-309 | Published online: 05 Sep 2013 Nutritional Neuroscience]. Epidemiological studies have indicated that dietary habits and the intake of antioxidants with diet can influence the incidence of neurodegenerative pathologies such as Alzheimer's disease and Parkinson's disease [De Rijk et al. "Dietary antioxidants and Parkinson disease. The Rotterdam Study. Arch Neurol. 1997 Jun;54(6):762-5; Engelhart M, et al. Diet and risk of dementia: Does fat matter?: The Rotterdam Study. Neurology. 2002 Dec 24;59(12):1915-21].

Lycopene, which is a liposoluble carotenoid present primarily in tomatoes and in red fruits, is characterised by a multitude of biological functions, such as inhibition of inflammation, and suppression of cellular carcinogenesis and tumour growth [Agca CA, et al. Lycopene counteracts the hepatic response to 7,12-dimethylbenz[a]anthracene by altering the expression of Bax, Bcl-2, caspases, and oxidative stress biomarkers. Pharm Biol. 2012 Dec;50(12):1513-8; [Trejo-Solis C, et al Multiple molecular and cellular mechanisms of action of lycopene in cancer inhibition. Evid Based Complement Alternat Med. 2013]. Various studies have shown that lycopene has a protective effect against the cerebral damage induced by oxidative stress. [Kaur H, et al. Neurochem Res. 2011 Aug; 36(8):1435-43. Protective effect of lycopene on oxidative stress and cognitive decline in rotenone induced model of Parkinson's disease; Di Matteo V, et al. Intake of tomato-enriched diet protects from 6-hydroxydopamine-induced degeneration of rat nigral dopaminergic neurons J Neural Transm Suppl. 2009;(73):333-41].

Astaxanthin is a carotenoid that is found in various marine microorganisms and animals, such as marine algae, salmon, trout and prawns [Higuera-Ciapara et al Crit Rev Food Sci Nutr. 2006;46(2):185-96 Astaxanthin: a review of its chemistry and applications]. It has been reported that astaxanthin prevents the cytotoxic damage induced by H₂O₂ in glioblastoma cells, reducing their secretion of pro-inflammatory cytokines [Speranza L, et al. Mar Drugs. 2012 Apr;10(4):890-9. Astaxanthin treatment reduced oxidative induced pro-inflammatory cytokines secretion in U937: SHP-1 as a novel biological target].

Bacopa monniera is a creeping herb studied for its pharmacological and therapeutic effects. The alcohol extract of Bacopa monniera contains a mixture of triterpene saponins, such as bacosides A and B [Chatterjee N, et al. (1963) Chemical examination of Bacopa munniera Wettst.: Part I - Isolation of chemical constituents. Indian J Chem,: 212-215]. Studies have shown that treatments with extracts of Bacopa monnieri protect the cells, *in vitro,* from the toxicity induced by oxidant substances [Manjeet Singh et al. Standardized extracts of bacopa monniera protect against MPP+- and paraquat-induced toxicity by modulating mitochondrial activities, proteasomal functions, and redox pathways. Toxicol Sci 2012 Jan 4;125(1):219-32]. In addition, Bacopa monnieri has shown, *in vitro,* effects of inhibition on the formation of free radicals and on cell damage [Russo A et al. Phytother Res. 2003 Sep;17(8):870-5. Free radical scavenging capacity and protective effect of Bacopa monniera L. on DNA damage].

Vitamin 12, also known as cobalamin, is a hydrosoluble vitamin that plays a key role in brain and nervous system function and in the formation of red blood cells [Aisen PS, et al Alzheimer Disease Cooperative Study.High-dose B vitamin supplementation and cognitive decline in Alzheimer disease: a randomized controlled trial. JAMA. 2008 Oct 15;300(15):1774-83]. Serum levels of vitamin B12 <250 micromol/L are associated with Alzheimer's disease and Parkinson's disease [Moore E, et al Int Psychogeriatr. 2012 Apr;24(4):541-56. Cognitive impairment and vitamin B12].

Vitamin B12 belongs to the class of coumarin compounds, which are characterised by various physiological and pharmacological activities, including anti-inflammatory, anti-bacterial and anti-oxidant activity. It has been demonstrated that vitamin B12 has neuroprotective effects against neuronal cell damage induced by H₂O₂ in undifferentiated SH-SY5Y cells.

The patent application US 2013/034530 relates to a nutrition supplement supporting healthy neural development, cognitive function, concentration, mood and behavioural stability and discloses a formulation containing vitamin B12 and Bacopa Monnieri.

The object of the present invention is to provide a combination and a composition alternative to those described in the prior art that is useful in the prevention and/or treatment of oxidative imbalances.

### SUMMARY OF THE INVENTION

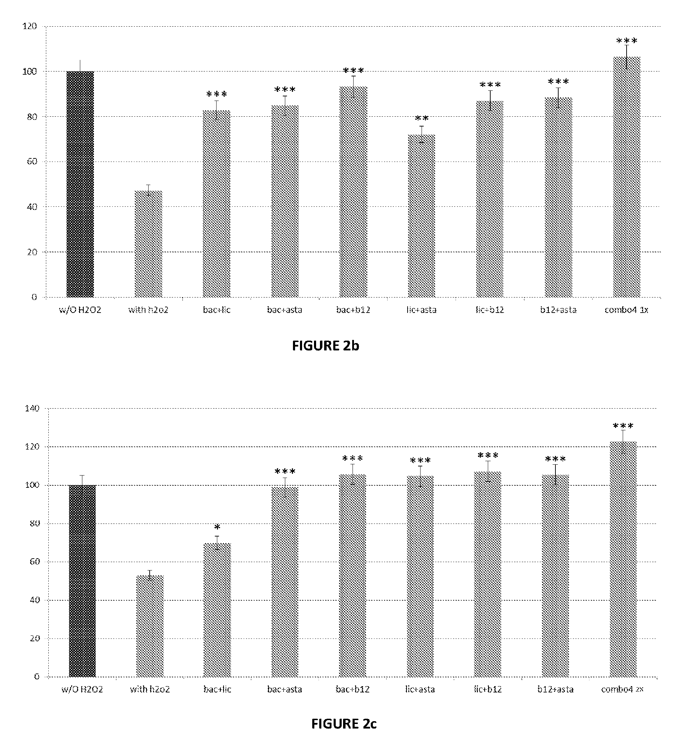

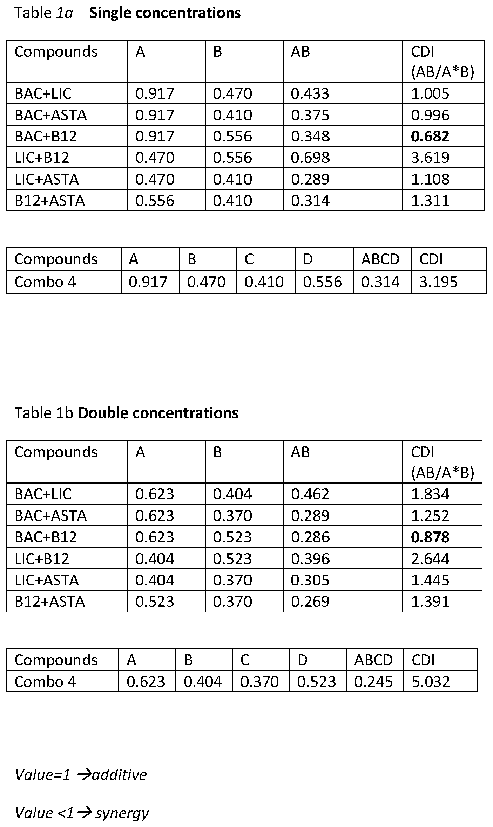

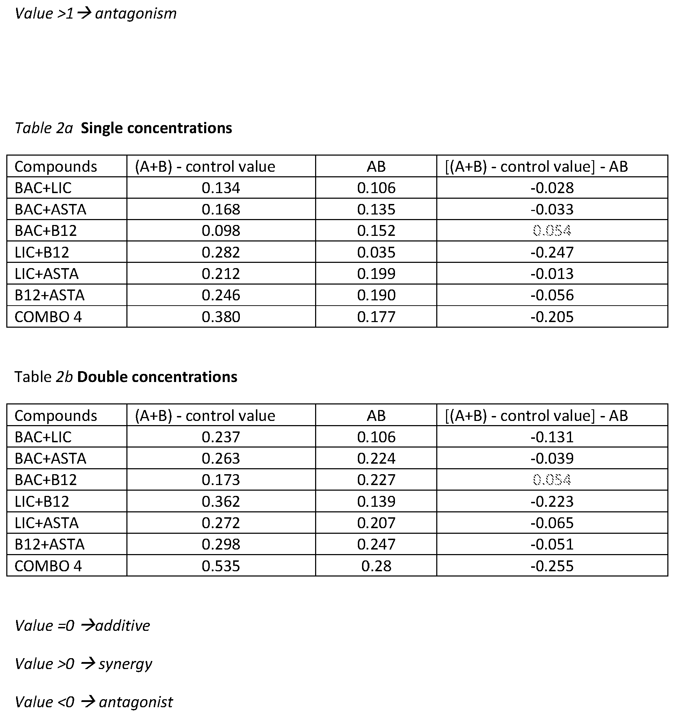

It has been found that nutraceutical, dietetic and nutritional compositions comprising extract of Bacopa and vitamin B12 are characterised by a surprising antioxidant activity that makes them particularly suitable for counteracting changes to the physiological redox balance. In particular, it has been found that differentiated neuronal cells (cell line SH-SY5Y) exposed to an oxidant insult (35 µM H₂O₂ for 2 hours), able to cause at least 40% cell death, if treated with mixtures of various antioxidant agents, such as Bacopa, vitamin B12, lycopene and/or astaxanthin, demonstrate significantly greater vitality compared to control cells (H₂O₂)) (fig. 2b e 2c). Surprisingly, the combination of two antioxidant compounds such as extract of Bacopa and vitamin B12 has demonstrated a synergistic effect as confirmed widely by mathematical models, known in the literature, used precisely for the purpose of distinguishing additive-type effects from synergistic effects (tables 1A, 1B, 2A and 2B).

A first subject of the present invention is therefore:
- A combination comprising an extract of Bacopa, astaxanthin, lycopene and vitamin B12, wherein said vitamin B12 is present in said composition in a dose between 2.00 and 5.00 microgram, said extract of Bacopa is present in said composition in a dose of 200 mg, said astaxanthin is present in said composition in a dose of 4 mg and said lycopene is present in said composition in a dose of 10 mg. A second subject of the invention is:
   A composition for oral use comprising a combination according to the present invention.

A third object of the invention is:
Non-therapeutic use of a combination comprising
a) an extract of Bacopa, preferably of Monnieri species; wherein said extract of Bacopa is present in said composition in a dose of 200 mg
b) vitamin B12, wherein said vitamin B12 is present in said composition in a dose between 2.00 and 5.00 microgram; and
c) lycopene and astaxanthin, wherein said astaxanthin is present in said composition in a dose of 4 mg and said lycopene is present in said composition in a dose of 10 mg or
of a composition according to any one of claims 5 to 7, to improve cognitive performance and/or improvement of mood and/or the state of psychological well-being.

Other advantages and features of the present invention will become clear from the following detailed description.

### DESCRIPTION OF THE DRAWINGS

FIGURE 1: differentiated and undifferentiated SH-SY5Y cells. The top images show phase-contrast microscopy. The bottom images show the immunelocalisation of B-III tubulin, which is a neuronal differentiation marker.
FIGURE 2a: treatment of differentiated SH-SY5Y with single oxidant at two different concentrations. Data provided as mean ± SE; 4 different tests performed in triplicate. **,p < 0.005; ***p < 0.0005
FIGURE 2b: combinations of 2 antioxidants and combinations of all 4 antioxidants (combo). In the combinations, the compounds were used at their lowest concentration (1X: lycopene 2 µM; Bacopa 3µg/ml; astaxanthin 12.5 µM; vitamin B12 0.3 µM.). Data provided as mean ± SE; 4 different tests performed in triplicate. **,p < 0.005; ***p < 0.0005
FIGURE 2c: combinations of 2 antioxidants and combinations of all 4 antioxidants (combo). In the combinations, the compounds were used at their highest concentration (2X: lycopene 4 µM; Bacopa 6 µg/ml; astaxanthin 25 µM; vitamin B12 0.6 µM). Data provided as mean ± SE; 4 different tests performed in triplicate. *,p < 0.01; ***p < 0.0005.

In the key to the drawings, the abbreviations have the following meanings:
bac= extract of Bacopa
Lico=lycopene
B12=vitamin B12
Ast=astaxanthin

### DETAILED DESCRIPTION OF THE INVENTION

The present invention describes a combination and a composition for oral use, in particular a nutraceutical, dietetic and nutritional composition comprising, as main ingredients with antioxidant effect, an extract of Bacopa, astaxanthin, lycopene and vitamin B12, wherein said vitamin B12 is present in said composition in a dose between 2.00 and 5.00 microgram, said extract of Bacopa is present in said composition in a dose of 200 mg, said astaxanthin is present in said composition in a dose of 4 mg and said lycopene is present in said composition in a dose of 10 mg..

In the present description, the term 'combination' means an association of the active ingredients both in the form of a physical mixture formed of said active ingredients in a single dosage unit, and in the form of a dosage unit physically separate from the active ingredient, but intended for administration simultaneously.

Bacopa is an aquatic plant belonging to the Plantaginacae family, known for its pharmacological and therapeutic effects. For the purposes of the present invention, the species Bacopa monnieri is preferably used.

The extract of Bacopa can be obtained by a person skilled in the art by means of conventional extraction techniques, for example, in water/alcohol or just alcohol. The alcohol used is preferably ethanol. The extract of Bacopa described here can be obtained from any portion of the plant known to contain bacosides. For example, the extraction can be performed using the leaves of Bacopa.

The extract of Bacopa comprised within the composition described here is preferably titrated between 10% and 30% in bacosides A and/or B, for example to 15%, 20%, or 25% in bacosides A and/or B. The composition of the invention also comprises vitamin B12 preferably in a dose between 2 and 5 micrograms, more preferably 5.

Vitamin B12 and methods for its procurement/synthesis are described in detail in the known prior art and therefore do not need to be discussed further at this juncture. By way of non-limiting example, vitamin B12 can be obtained as described in J.G. LeBlanc et al. Journal of Applied Microbiology ISSN 1364-5072.

The composition also comprises lycopene and astaxanthin.

Lycopene is a hydrocarbon belonging to the group of carotenoids. The primary source of lycopene is the tomato. Lycopene can be extracted from ripe tomatoes, in particular, using an ethereal solvent (Susanne Rath, et al. Lycopene Extract from Tomato, Chemical and Technical Assessment (CTA). Available online at http.//www.fao.org/fileadmin/templates/agns/pdf/jec fa/cta/71/lycopene_extract_from_tomato.pdf. 2009). Lycopene can be of natural or synthetic origin. Synthetic lycopene is obtained for example using a condensation reaction, known as the Wittig reaction. The process for obtaining synthetic lycopene is described for example in the article *Lycopene (synthetic) (CTA) 2006* di Zofia Olempska-Beer. Synthetic lycopene can be obtained also by means of purification from microorganisms suitable for the production of this substance.

In accordance with one embodiment, because lycopene is insoluble in water, it is preferably dispersed in matrices before being used in the compositions described herein, for example is solubilised in formulations in edible fats and oils, in emulsions, or hydro-dispersible powders.

The lycopene can be present in the composition in the form of an extract of lycopene titrated between 5% and 15%, preferably to 10%.

The extract of lycopene, as titrated above, is present in the composition in a dose of 10 mg.

The composition described herein also comprises astaxanthin. Astanxanthin is present in marine microorganisms and animals. By way of non-limiting example, astaxanthin can be obtained as described in Ranga Rao Ambati et al. Mar. Drugs 2014, 12, 128-152. The astaxanthin is present in the composition described herein in a dose of 4 mg.

The compositions according to the present invention can be formulated in various ways, for example as capsules, soft capsules, tablets, pills, jellies, powders or granules. These formulations shall comprise vitamin B12, the extract of Bacopa and any other antioxidant ingredients, such as lycopene and/or astaxanthin and one or more excipients acceptable for oral administration.

Such excipients can be selected for example from those normally known in the prior art and include, but are not limited to: a) carriers, such as sodium citrate and calcium phosphate; b) fillers, such as amide, lactose, microcrystalline cellulose, sucrose, glucose, mannitol and colloidal silica; c) humectants, such as glycerol; d) disintegrants, such as calcium carbonate, amides, amide derivatives, cellulose derivatives, and polyvinylpyrrolidone derivatives, sodium silicates and sodium carbonate; e) binders, such as carboxymethylcellulose, alginates, gelatin, polyvinylpyrrolidone, sucrose, polymeric derivatives of cellulose, amide derivatives; f) retarders, such as paraffin, cellulose polymers, fatty acid esters; g) absorption accelerators, such as quaternary ammonium compounds; h) wetting agents and surfactants, such as cetyl alcohol and glycerol monostearate; i) adsorbents, such as clay bentonite and kaolin; k) lubricants, such as talc, calcium stearate, magnesium stearate, polyethylene glycol, sodium lauryl sulphate, sodium stearyl fumarate; j) anti-friction agents, such as talc and colloidal silica.

The solid dosage forms, such as tablets, capsules, soft capsules, gelatins, pills and granules, can be coated by enteric, gastric or another type of coating known in the art. They can contain opacifying agents and can be of the type allowing active ingredient release only, or preferably, within a certain section of the intestine, possibly in a delayed manner. Substances that can allow such delayed use include, but are not limited to, polymers and waxes.

Soft capsules can accommodate the antioxidant active substances in liquid form alone or in solutions, suspensions or emulsions of the active substances in a liquid solvent. Soft capsules can be characterised by a casing that has qualities similar to those of rigid casings, but is thicker and soft.

Liquid forms suitable for oral administration are, for example, emulsions, solutions or suspensions, either prepared or extemporary, syrups and elixirs. Excipients suitable for the formulations according to the present invention in liquid form for oral use include, but are not limited to, diluents, such as water or other solvents, solubilisers and emulsifiers selected from ethyl alcohol, polyalcohols, propylene glycol, glycerol, polyethylene glycol and sorbitan esters. These formulations can also contain sweeteners and flavourings.

The compositions shall be selected for example from a nutraceutical, dietetic or nutritional composition, a food product, a beverage, a dietary supplement, a nutraceutical, a medicament, a medicated food, a food for specific medical purposes, or a food. The compositions shall be intended primarily for use by human beings, but could also be used in animals.

The compositions according to the present invention can comprise one or more suitable food and/or preserving and/or acidifying and/or antioxidant and/or immunostimmulant and/or colouring and/or probiotic and/or prebiotic ingredients.

The combination of the invention comprising
a) an extract of Bacopa, preferably of the monnieri species, wherein said extract of Bacopa is present in said composition in a dose of 200 mg
b) vitamin B12 wherein said vitamin B12 is present in said composition in a dose between 2.00 and 5.00 microgram; and
c) lycopene and astaxanthin, wherein said astaxanthin is present in said composition in a dose of 4 mg and said lycopene is present in said composition in a dose of 10 mg;

or a combination of a composition according to the present invention,
can be used, administered or ingested to improve cognitive performance and/or improve mood and/or the state of psychological well-being.

The term "cognitive performance" means the performance relating to all processes such as memory, association, concept formation, language, attention, perception, and action. As known, "cognitive performance" tends to reduce with age, without this reduction being classifiable as pathological. It is in the sense of its nonpathological meaning that the term "cognitive performance" is meant here.

The composition described herein can also be used to restore and/or improve mood where mood changes are not classifiable as pathological.

### EXPERIMENTAL SECTION

### Materials and methods

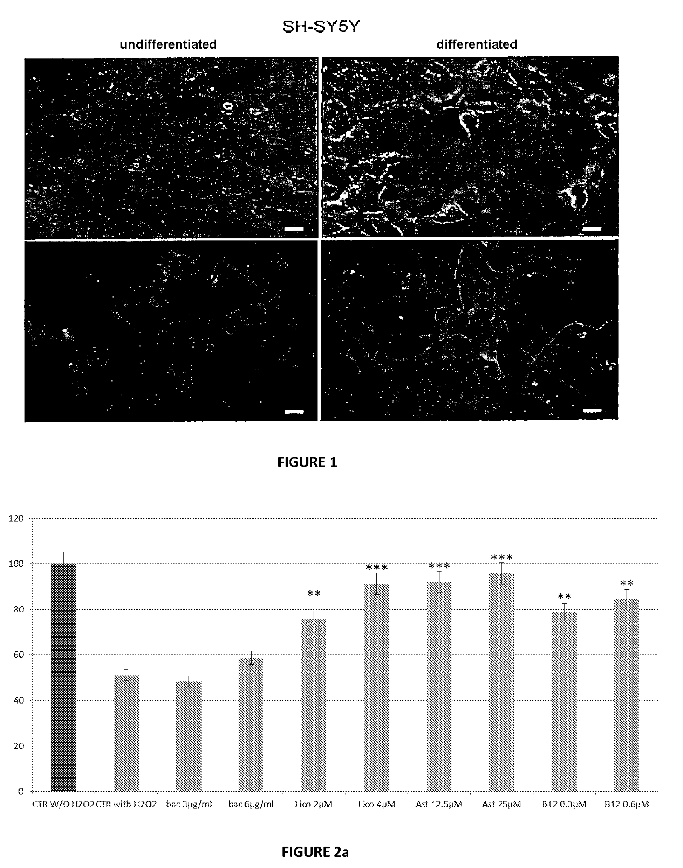

*Experimental procedure.* The human neuroblastoma cell line SH-SY5Y, which differentiates into neuronallike cells, was used as *in vitro* model. This cell line is generally accepted as a human neuronal model and is commonly used for the study of neurodegenerative diseases (Cimini A, et al Cell Biochem. 2013;114(10):2209-20; Song G, et al Mol Med Rep. 2015 Nov;12(5):7615-22; Cimini A, et all Acta Biomater. 2012 Jul;8(6):2056-67; Cimini A, et al J Cell Biochem. 2013 Mar;114(3):708-15; Chakravarthy B, et all. J Alzheimers Dis. 2010;19(3):915-25; Grimm MO et al Int J Mol Sci. 2016 Oct 29;17(11); Liu J, et al Neurochem Res. 2016 Sep;41(9):2311-23; Liu Y et al. Mol Med Rep. 2017 Jan;15(1):285-290). The cells were plated at a density of 1×104 cells/cm² and cultivated in culture medium RPMI 1640 in the absence of serum and containing differentiation factor N2 for the purpose of promoting neuronal differentiation (Fig 1).

The cytoprotective effects of different antioxidants were assessed after induction of oxidative stress with H₂O₂.

After studying the concentration of H₂O₂ and the oxidative stress conditions to be induced experimentally, the concentration of 35 µM H₂O₂ for 2 hours was selected, with the objective of achieving at least 40% cell death. Following the oxidant insult, the differentiated SH-SY5Y cells were treated for 24 hours with 4 different antioxidant compounds administered as single treatment or in different combinations. The antioxidants used were: extract of Bacopa, vitamin B12, lycopene and astaxanthin.

The antioxidants were prepared by diluting the powder in culture medium RPMI 1640 without serum, and two concentrations were selected for each oxidant. In particular, the concentrations used were:
- lycopene 2 µM (single concentration) or 4 µM (double concentration);
- Bacopa monnieri 3µg/ml (single concentration) or 6 µg/ml (double concentration);
- astaxanthin 12.5 µM (single concentration) or 25 µM (double concentration);
- vitamin B12 0.3 µM (single concentration) or 0.6 µM (double concentration).

### Vitality and cell mortality

Control cells (cells treated only with H₂O₂) and treated cells (cells treated with H₂O₂ and then antioxidant) were plated in a 96-well plate, where they were incubated, after treatment, for 2 h with CellTiter 96 Aqueous One Solution for the purpose of application of a colorimetric method based on 3-(4,5-dimethylthiazol-2-yl)-5-(3-carboxymethoxy phenyl)-2-(4-sulfophenyl)-2H-tetrazol (MTS). The amount of formazan produced, which is an indicator of cell vitality, was measured at 490 nm using an ELISA plate reader. All of the MTS assays were performed in triplicate.

### Statistical analysis

For the statistical analyses, the samples were processed and analysed using the Student's t-test. The experiments were performed at least in triplicate. The data were expressed as mean ± standard error. P values less than 0.05 were considered to be statistically significant.

For the purpose of stabilising possible synergistic effects between the various combinations of tested antioxidants, the values relating to the amount of formazan produced (indicator of cell vitality), obtained at 490 nm with Elisa reader, were analysed by applying the following mathematical equations:

### 1) Data shown in table 1

CDI (coefficient of drug interaction; coefficient of interaction between the molecules)=AB/(A*B) where A and B indicate the values of formazan produced following the treatment with the single ingredients and AB indicates the value obtained from the combination of two of said ingredients. The concentrations are defined in the paragraph above ("experimental procedure").
CDI <1 indicates synergistic effect
CDI =1 indicates additive effect
CDI >1 indicates antagonistic effect

### 2) Data shown in table 2

If the result is = 0 additive effect >0 synergistic effect <0 antagonistic effect (Klaassen, C., 2001. "Casarett and Doull's Toxicology: The Basic Science of Poisons. 6th EditionSoica C, Oprean C, Borcan F, Danciu C, Trandafirescu C, Coricovac D, Cr 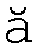 iniceanu Z, Dehelean CA, Munteanu M The synergistic biologic activity of oleanolic and ursolic acids in complex with hydroxypropyl-γ-cyclodextrin. Molecules. 2014 Apr 17;19(4):4924-40).

Using both the equations, the results observed were the same for each combination tested.

### Results

Cell vitality, assessed by means of MTT assay, in treated cells and in control cells is shown in Figure 2.

The vitality of the cells exposed for 2 h to 35 µM H₂O₂ is significantly lower compared to the control cells, approximately 50% less.

Treatment of the cells with the single antioxidant compound improves cell survival, moreover to a greater extent with a higher used concentration. In particular, lycopene 4 µM, astaxanthin 25 µM and vitamin B12 0.6 µM are able to bring the cell vitality back to values almost comparable to those of the control cells (the latter not having been treated with hydrogen peroxide) (Fig. 2a)

Surprisingly, as indicated by the results reported in tables 1 and 2 (a and b), it was observed that the combination of the two antioxidant compounds Bacopa + vitamin B12 shows a synergistic effect at both concentrations used. The synergistic effect, by contrast, was not observed for the other combinations tested.

In addition, it was observed that the combination with all 4 antioxidant compounds shows a greater vitality compared to that observed in the control cells (Fig 2b and 2c).

### Study in vivo:

### recruitment protocol

The participants were recruited from subjects aged 60 years and above. The inclusion criteria stipulated, amongst other things, the exclusion of subjects with cardiovascular diseases and with cerebrovascular events, diagnosed dementia or other neurological diseases, thyroid disorders or inflammatory diseases. In addition, for the purpose of preventing any alterations to the test for the assessment of the cognitive performance on account of concomitant depression, subjects with a score based on the geriatric depression scale >11 were excluded. Smokers, habitual users of antioxidant supplements (including vitamins C and E), habitual consumers of chocolate or other cacao-based products (daily consumption of any amount), and individuals prescribed medicaments known to have antioxidant properties (including statins and glitazones) or known to interfere with cognitive function (including benzodiazepines and anti-depressants) were all excluded as participants in the study.

### Study design and results

The study was a double-blind study lasting 4-12 weeks. The study was randomised and had parallel arms (control with placebo and subjects treated with the mixture of the invention). After recruitment the following steps were performed: the daily food habits of the participants were evaluated, any nutritional insufficiencies were corrected, and the introduction of test products at low dietetic content into the daily diet was discussed. The participants were educated so as to maintain their usual lifestyle, fruit and vegetable consumption, avoiding, as far as possible, eating and drinking food and beverages containing specific flavanols, including tea, red wine, vegetable and fruit juices and chocolate. To this end, each participant was provided with a comprehensive list of foods. All of the participants were encouraged to continue with their daily physical activities for the period of the study. After a period of enrolment of approximately one week, the participants were assigned randomly to two groups (intake of the placebo or intake of the antioxidant composition described herein), thus taking once per day, for a period between 4 and 12 weeks, either the control composition (placebo) or a composition as detailed in the table above. For the purpose of maximising the *compliance* with the treatment and minimising lifestyle changes and diet changes, weekly monitoring was performed by telephone contact or pre-arranged meetings, according to the needs of the participants.

At intervals of 2, 4, 6, 8, 10 and 12 weeks after initial administration, changes in the cognitive function, mood, and self-perception of well-being were assessed. The following were also assessed: blood pressure, metabolic changes, and plasma markers of oxidative stress.

**Table - composition taken by the participants**

| **INGREDIENTS** | **DOSE** |
|---|---|
| Extract of Bacopa monnieri | *200 mg* circa (extract titrated to 20% in bacosides) |
| Vitamin B12 | *5 micrograms* (approx..) |
| Astaxanthin | *4 mg* (approx..) |
| Extract of lycopene | *10 mg* (approx..) (titrated to 5%) |

### Assessment of cognitive function

The cognitive assessments were performed both at the start and after 4, 8, 10 and/or 12 weeks (± 2 days) using a combination of 4 well-validated, standard tests: Mini Mental State Examination (MMSE), Trail Making Test A (TMTA), Trail Making Test B (TMTB), and verbal fluidity test (VFT). The MMSE test is based on a video means widely used for the assessment of cognitive deficiency. In particular, MMSE covers five areas of cognitive function, including orientation, attention, calculation, memory and language with scores varying from 0 to 30. TMT, which explores the visual-conceptual and motor-visual functions, is frequently used as a neuropsychological test for sensitivity to cerebral damage. This test consists of two parts: TMT-A and TMT-B. TMT-A is a visual assessment test and requires the subject to draw a line connecting consecutive numbers. TMT-B adds cognitive flexibility to TMT-A and requires the subject to draw a line connecting numbers and letters in alternate sequence. The score is given by the time taken in seconds to complete the test. To assess verbal fluidity, the participants are asked to list as many names as possible that start with a given letter. The individual scores are converted into standardised points (z-score) based on the averages (and the standard deviations) of the entire population at the start of the study (at baseline). In order to assess cognitive performance, the Montreal Cognitive Assessment (MoCA) was also used. The MoCA measures seven cognitive areas, including cognitive function and abstraction.

*Assessment of mood and of self-perception of well-being.*

Mood was then evaluated using the "Profile of Mood Stated" (POMS), which is one of the most widely used tools. Higher scores reflect a decline in mood, except for the subscale of vigour, where higher scores reflect improved mood.

Psychological well-being was assessed using "General Health Questionnaire" (GHQ-12), an indicator revealing the severity of psychological morbidity.

### Breadth of the sample

The study was based on an estimated sample of approximately 60 subjects, which was deemed to be a sufficient number to reach 95% confidence of revealing a moderate effect (Cohen d=0.25) with 1 degree of freedom and an alpha of 0.05 for the z-score between treatment and control.

### Preliminary results

The study above confirmed an improvement in cognitive performance, mood and/or psychological well-being following ingestion for 4, 8 and 12 weeks of the mixture of the four above-described antioxidants.

## Claims

1. A combination comprising an extract of Bacopa, astaxanthin, lycopene and vitamin B12, wherein said vitamin B12 is present in said composition in a dose between 2.00 and 5.00 microgram, said extract of Bacopa is present in said composition in a dose of 200 mg, said astaxanthin is present in said composition in a dose of 4 mg and said lycopene is present in said composition in a dose of 10 mg.

2. The combination according to claim 1, wherein said extract is obtained from the species *Bacopa Monnieri.*

3. The combination according to claim 1 or 2 wherein said extract of Bacopa is titrated between 10 and 30% in Bacosidi, preferably to 20% by Bacosidi.

4. The combination according to any one of claims 1 to 3, wherein said lycopene is an extract of lycopene titrated between 5% and 15%

5. A composition for oral use comprising a combination according to any one of the claims 1 to 4.

6. The composition according to claim 5 in the formulation selected between capsule, soft capsule, tablet, pill, jelly, powder, granule, emulsion, solution, syrup.

7. The composition according to claims 5 to 6 wherein said composition is a nutraceutical composition, dietary and nutritional a foodstuff, a beverage, a food supplement, a nutraceutical, a medicament, a food or a medicated food for special medical purposes.

8. Non-therapeutic use of a combination comprising
a) an extract of Bacopa, preferably of Monnieri species; wherein said extract of Bacopa is present in said composition in a dose of 200 mg
b) vitamin B12, wherein said vitamin B12 is present in said composition in a dose between 2.00 and 5.00 microgram; and
c) lycopene and astaxanthin, wherein said astaxanthin is present in said composition in a dose of 4 mg and said lycopene is present in said composition in a dose of 10 mg or of a composition according to any one of claims 5 to 7, to improve cognitive performance and/or improvement of mood and/or the state of psychological well-being.

## Patentansprüche

1. Kombination, umfassend einen Extrakt aus Bacopa, Astaxanthin, Lycopin und Vitamin B12, wobei das Vitamin B12 in der Zusammensetzung in einer Dosis zwischen 2,00 und 5,00 Mikrogramm vorliegt, der Extrakt aus Bacopa in der Zusammensetzung in einer Dosis von 200 mg vorliegt, das Astaxanthin in der Zusammensetzung in einer Dosis von 4 mg vorliegt und das Lycopin in der Zusammensetzung in einer Dosis von 10 mg vorliegt.

2. Kombination nach Anspruch 1, wobei der Extrakt aus der Spezies *Bacopa Monnieri* gewonnen wird.

3. Kombination nach Anspruch 1 oder 2, wobei der Extrakt von Bacopa zwischen 10 und 30 % in Bacosiden, vorzugsweise zu 20 % durch Bacosiden, titriert ist.

4. Kombination nach einem der Ansprüche 1 bis 3, wobei das Lycopin ein Extrakt aus Lycopin ist, der zwischen 5 % und 15 % titriert ist.

5. Zusammensetzung für eine orale Verwendung, umfassend eine Kombination nach einem der Ansprüche 1 bis 4.

6. Zusammensetzung nach Anspruch 5 in der Formulierung, ausgewählt aus Kapsel, Weichkapsel, Tablette, Pille, Gelee, Pulver, Granulat, Emulsion, Lösung, Sirup.

7. Zusammensetzung nach den Ansprüchen 5 bis 6, wobei die Zusammensetzung eine nutrazeutische Zusammensetzung, ein diätetisches und nutritives Nahrungsmittel, ein Getränk, ein Nahrungsergänzungsmittel, ein Nutrazeutikum, ein Arzneimittel, ein Lebensmittel oder ein medizinisches Lebensmittel für besondere medizinische Zwecke ist.

8. Nichttherapeutische Verwendung einer Kombination umfassend
a) einen Extrakt aus Bacopa, vorzugsweise der Spezies Monnieri; wobei der Extrakt aus Bacopa in der Zusammensetzung in einer Dosis von 200 mg vorliegt
b) Vitamin B12, wobei das Vitamin B12 in der Zusammensetzung in einer Dosis zwischen 2,00 und 5,00 Mikrogramm vorliegt; und
c) Lycopin und Astaxanthin, wobei das Astaxanthin in der Zusammensetzung in einer Dosis von 4 mg vorliegt und das Lycopin in der Zusammensetzung in einer Dosis von 10 mg oder aus einer Zusammensetzung nach einem der Ansprüche 5 bis 7, um die kognitive Leistungsfähigkeit und/oder eine Verbesserung der Stimmung und/oder des Zustands des psychischen Wohlbefindens zu verbessern, vorliegt.

## Revendications

1. Combinaison comprenant un extrait de Bacopa, de l'astaxanthine, du lycopène et de la vitamine B12, dans laquelle ladite vitamine B12 est présente dans ladite composition à une dose comprise entre 2,00 et 5,00 microgrammes, ledit extrait de Bacopa est présent dans ladite composition à une dose de 200 mg, ladite astaxanthine est présente dans ladite composition à une dose de 4 mg et ledit lycopène est présent dans ladite composition à une dose de 10 mg.

2. Combinaison selon la revendication 1, dans laquelle ledit extrait est obtenu à partir de l'espèce *Bacopa Monnieri.*

3. Combinaison selon la revendication 1 ou 2, dans laquelle ledit extrait de Bacopa est titré entre 10 et 30 % en bacosides, de préférence à 20 % en bacosides.

4. Combinaison selon l'une quelconque des revendications 1 à 3, dans laquelle ledit lycopène est un extrait de lycopène titré entre 5 % et 15 %

5. Composition pour une utilisation par voie orale, comprenant une combinaison selon l'une quelconque des revendications 1 à 4.

6. Composition selon la revendication 5, sous la formulation choisie parmi capsule, capsule molle, comprimé, pilule, gelée, poudre, granulé, émulsion, solution, sirop.

7. Composition selon les revendications 5 à 6, dans laquelle ladite composition est une composition nutraceutique, diététique et nutritionnelle, une denrée alimentaire, une boisson, un complément alimentaire, un produit nutraceutique, un médicament, un aliment ou un aliment médicamenteux destiné à des fins médicales spéciales.

8. Utilisation non thérapeutique d'une combinaison comprenant
a) un extrait de Bacopa, de préférence de l'espèce Monnieri ; dans laquelle ledit extrait de Bacopa est présent dans ladite composition à une dose de 200 mg
b) de la vitamine B12, dans laquelle ladite vitamine B12 est présente dans ladite composition à une dose comprise entre 2,00 et 5,00 microgrammes ; et
c) du lycopène et de l'astaxanthine, dans laquelle ladite astaxanthine est présente dans ladite composition à une dose de 4 mg et ledit lycopène est présent dans ladite composition à une dose de 10 mg ou d'une composition selon l'une quelconque des revendications 5 à 7, pour améliorer les performances cognitives et/ou pour l'amélioration de l'humeur et/ou de l'état de bien-être psychologique.
